# EUROPÄISCHE PATENTANMELDUNG

(11) **EP 2 589 439 A1**
(43) Veröffentlichungstag der Anmeldung: **08.05.2013**
(21) Anmeldenummer: 12007168.3
(22) Anmeldetag: 16.10.2012
(51) Int. Cl.: B05D 1/28, B05D 1/24

(54) **Beschichtungsverfahren und Beschichtungsvorrichtung für medizinische Implantate**

(30) Priorität: 03.11.2011 DE 102011117526
(71) Anmelder: Heraeus Medical GmbH, 61273 Wehrheim (DE)
(72) Erfinder: Vogt, Sebastian, 99092 Erfurt (DE); Copanaki, Ekaterini, 60322 Frankfurt (DE); Gaiser, Sebastian, 60325 Frankfurt (DE)
(74) Vertreter: Panten, Kirsten

(57) **Zusammenfassung**

Die Erfindung betrifft Verfahren zur zumindest bereichsweisen Beschichtung eines medizinischen Implantats, vorzugsweise eines künstlichen Gelenks oder einer Fixierung für ein Gelenk, bei dem
ein medizinisches Implantat bereitgestellt wird,
eine Flüssigkeit umfassend zumindest eine pharmazeutisch aktive Substanz in eine Vorrichtung umfassend ein zumindest bereichsweise komprimiertes, elastisches, poröses Übertragungsmittel eingefüllt wird,
das Volumen des Übertragungsmittels vergrößert wird, wobei die Flüssigkeit zumindest teilweise in die Poren des Übertragungsmittels aufgenommen wird und
anschließend die Flüssigkeit vom Übertragungsmittel auf eine zu beschichtende Oberfläche des medizinischen Implantats übertragen wird.

Die Erfindung betrifft auch eine Vorrichtung zur zumindest bereichsweisen Beschichtung eines medizinischen Implantats, insbesondere mit einem Verfahren nach einem der vorangehenden Ansprüche, wobei die Vorrichtung zumindest ein zumindest bereichsweise komprimiertes, elastisches, poröses Übertragungsmittel umfasst.

## Beschreibung

Die Erfindung betrifft ein Verfahren zur zumindest bereichsweisen Beschichtung eines medizinischen Implantats, vorzugsweise eines künstlichen Gelenks oder einer Fixierung für ein Gelenk.

Die Erfindung betrifft auch eine Vorrichtung zur zumindest bereichsweisen Beschichtung eines medizinischen Implantats, insbesondere mit einem solchen Verfahren.

Die Beschichtung von medizinischen Implantaten mit pharmazeutischen Wirkstoffen hat in den letzten Jahren zunehmend Beachtung gefunden. Eine Hauptanwendung von Beschichtungsverfahren liegt dabei im antibiotischen Schutz der Oberfläche von Implantatmaterialien. Daneben ist auch die Beschichtung mit Wachstumsfaktoren, Gerinnungsfaktoren, Bisphosponaten, Anästhetika, Proteinen, Peptiden, Hormonen und weiteren Wirkstoffen von Interesse.

Die Implantation von Gelenkendoprothesen und auch von Osteosynthesematerialien ist immer mit einer gewissen Gefahr einer mikrobiellen Kontamination verbunden. Wenn es mikrobiellen Keimen gelingt, sich an der Implantatoberfläche anzusiedeln, kann es zur Ausbildung einer post-operativen Osteitis/Osteomyelitis kommen. Die Osteitis/Osteomyelitis stellt eine schwerwiegende Komplikation für den Patienten dar, die zusätzlich mit erheblichen Kosten verbunden ist.

Seit Jahrzehnten wird mit klinischem Erfolg bei zementierten Gelenkendoprothesen mit Gentamicin dotierter PMMA-Knochenzement verwendet. Das im Knochenzement enthaltende Breitbandantibiotikum Gentamicin schützt die Oberfläche des Knochenzementes wirksam gegen bakterielle Infektionen.

Bei nicht-zementierten Gelenkendoprothesen und bei Osteosynthesematerialien wurde eine Reihe von Lösungswegen vorgeschlagen, um ebenfalls einen lokalen antibiotischen Schutz der Implantatoberflächen zu erreichen.

So wurde in mehreren Patentschriften die Verwendung von in Wasser gering löslichen Antibiotika-Salzen beschrieben. Exemplarisch seien dafür die EP 0 623 349 A1, EP 1 470 829 A1, EP 1 374 923 A2, DE 101 42 465 A1 und DE 44 04 018 A1 genannt. Diese in Wasser gering löslichen Salze lösen sich unter Freisetzung der enthaltenen Antibiotika durch Einwirkung von Körperflüssigkeiten auf. Vorteilhaft ist die protrahierte Wirkstofffreisetzung. Nachteilig ist jedoch die aufwendige Herstellung dieser Salze.

Alternativ dazu ist es auch möglich, in Wasser lösliche Antibiotika-Salze zu verwenden. Das Problem liegt dabei darin, das Antibiotikum auf der Implantatoberfläche zu fixieren.

Die Mehrzahl der bisherigen beschriebenen Beschichtungen ist vorzugsweise für die Fertigung von beschichteten Implantaten unter industriellen Bedingungen bestimmt. Das bedeutet, dass die industrielle Beschichtung dieser Implantate nur mit wenigen für die Großanwendung relevanten Wirkstoffen erfolgen kann, um eine wirtschaftliche industrielle Fertigung mit entsprechenden hohen Stückzahlen gewährleisten zu können.

Insbesondere bei antibiotischen Beschichtungen ist es jedoch im Hinblick auf die sich zunehmend problematisch gestaltende Resistenzsituation und dem dabei vermehrten Auftreten von multiresistenten Keimen, wie MRSA und MRSE, von Interesse, genau auf die jeweiligen Keime abgestimmte Antibiotika oder Antibiotika-Kombinationen zur Beschichtung von Revisionsprothesen im Zuge des einzeitigen oder zweizeitigen septischen Gelenkprothesenwechsels einzusetzen, um einen initialen antibiotischen Schutz der Implantatoberflächen zu gewährleisten.

Nachteilig ist hieran, dass die Verfahren zur Beschichtung der medizinischen Implantate relativ aufwendig sind. Eine variable kurzfristige Anwendung ist nicht möglich. Es müssen für verschiedene Situationen verschiedene beschichtete medizinische Implantate bereitgehalten werden, um den Bedürfnissen der unterschiedlichen Patienten gerecht zu werden. Dies erfordert eine umfangreiche Lagerhaltung und verhindert ungewöhnliche Lösungen für spezielle Fälle.

Die nicht vorveröffentlichte DE 10 2010 055 559 befasst sich mit einem Beschichtungsverfahren und einer Beschichtungsvorrichtung, bei der ein Übertragungsmittel in einem Gefäß eingesetzt wird, wobei das Übertragungsmittel vor dem Beschichten eines medizinischen Implantats mit einer Flüssigkeit beinhaltend zumindest eine pharmazeutisch aktive Substanz getränkt wird. Nachteilig ist hieran, dass eine große Menge der Flüssigkeit in das Gefäß gegeben werden muss, damit das Übertragungsmittel gut gefüllt wird. Dies betrifft insbesondere Flüssigkeiten, die keine hohe Adhäsion mit der Oberfläche des Übertragungsmittels haben oder Flüssigkeiten mit sehr teuren Substanzen oder mit Substanzen die die Umwelt, die Umgebung und/oder insbesondere den Operationssaal kontaminieren können.

Die Aufgabe der Erfindung besteht also darin, die Nachteile des Stands der Technik zu überwinden. Insbesondere soll ein einfaches und leicht anzuwendendes Verfahren und eine Vorrichtung hierfür bereitgestellt werden, mit denen eine medizinische Prothese beschichtet werden kann, ohne dass dadurch der Ablauf einer Operation (OP) gestört wird. Es sollen möglichst viele verschiedene medizinische Implantate mit dem Verfahren und der Vorrichtung beschichtet werden können. Auch sollen das Verfahren und die Vorrichtung variabel einsetzbar sein, so dass sie an die medizinischen Notwendigkeiten, insbesondere an eine für den Patienten geeignete Medikamentierung anpassbar sind. Der in Operationssälen gebotenen Reinheit soll ebenfalls Rechnung getragen werden. Ferner soll die Umwelt und die oftmals kostbaren Ressourcen geschont werden.

Die Aufgabe der Erfindung besteht ferner darin, ein möglichst einfaches Beschichtungsverfahren zu entwickeln, das vom OP-Personal während einer OP mit geringstem Zeitaufwand zum Beschichten von unterschiedlichsten Implantaten, die von beliebigen Herstellern produziert sein können, mit pharmazeutischen Zubereitungen eingesetzt werden kann. Weiterhin ist es eine Aufgabe der Erfindung, eine einfache Beschichtungsvorrichtung zu entwickeln, die es dem OP-Personal erlaubt, unter OP-Bedingungen Implantate mit geringstem Aufwand unter Verwendung beliebiger flüssiger oder fließfähiger pharmazeutischer Zubereitungen, wie Flüssigkeiten oder Suspensionen zu beschichten. Die Vorrichtung soll weiterhin so gestaltet sein, dass möglichst keine Sprühnebel oder Tropfen den OP-Bereich kontaminieren können. Ferner ist es auch Aufgabe der Erfindung, dass durch das Verfahren oder die Vorrichtung möglichst keine Kunststoffpartikel, Metallpartikel oder andere nicht oder nur teilweise biodegradierbare Partikel freigesetzt werden. Ebenso soll auch die Freisetzung von Partikeln von Bürsten, Pinseln oder anderer Haare, Fasern oder Borsten vermieden werden. Eine weitere Aufgabe besteht darin, dass die Vorrichtung besonders zur Beschichtung von nicht-zementierten Gelenkendoprothesen und Osteosynthesematerialien geeignet sein soll.

Die Aufgaben der Erfindung und weitere nicht genannte Probleme werden durch ein Verfahren zur zumindest bereichsweisen Beschichtung eines medizinischen Implantats, vorzugsweise eines künstlichen Gelenks oder einer Fixierung für ein Gelenk gelöst, bei dem ein medizinisches Implantat bereitgestellt wird, eine Flüssigkeit umfassend zumindest eine pharmazeutisch aktive Substanz in eine Vorrichtung umfassend ein zumindest bereichsweise komprimiertes, elastisches, poröses Übertragungsmittel eingefüllt wird, das Volumen des Übertragungsmittels vergrößert wird, wobei die Flüssigkeit zumindest teilweise in die Poren des Übertragungsmittels aufgenommen wird und anschließend die Flüssigkeit vom Übertragungsmittel auf eine zu beschichtende Oberfläche des medizinischen Implantats übertragen wird.

Erfindungsgemäße Verfahren erfolgen vor dem Einsetzen der medizinischen Implantate. Die Verfahren finden also "ex vivo" statt.

Unter einer pharmazeutisch aktiven Substanz sind erfindungsgemäß pharmazeutisch wirksame Mittel und Mittel zu verstehen, die pharmakologisch wirken, sowie solche, die eine pharmakologische Wirkung unterstützen oder sonst in irgendeiner Weise die Selbstheilungskräfte des Körpers unterstützen. Beispiele sind hierfür Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen.

Erfindungsgemäß kann auch vorgesehen sein, dass die Flüssigkeit eine wässrige Lösung eines Antibiotikums umfasst, bevorzugt eine wässrige Gentamicinsulfat-Lösung mit einem Gentamicinsulfat-Gehalt von 10,0 bis 88,0 Gewichtsprozent verwendet wird, wobei ganz besonders eine Gentamicinsulfat-Lösung mit einem Gentamicinsulfat-Gehalt von 75,0 bis 80,0 Gewichtsprozent bevorzugt verwendet wird. Diese Gentamicinsulfat-Lösung hat eine ölig-viskose Konsistenz und haftet sehr gut auf Metalloberflächen.

Es kann dabei ferner vorgesehen sein, dass pharmazeutisch übliche Stabilisatoren in den Gentamicinsulfat-Lösungen enthalten sind. Diese verbessern die Haltbarkeit und damit die Verwendbarkeit der aufzutragenden Flüssigkeit.

Erfindungsgemäß kann auch die Verwendung von anderen Aminoglykosid-Antibiotika-Lösungen, wie wässrige Lösungen des Tobramycinsulfats, des Amikacinsulfats, des Netilmicinsulfats und des Sisomycinsulfats als Flüssigkeit oder Flüssigkeitsbestandteile vorgesehen sein. Es ist auch möglich, wässrige Lösungen des Vancomycins, des Dalbavancins, des Ramoplanins, des Daptomycins, des Moxifloxacins, des Clindamycins und des Lincomycins einzusetzen.

Ferner kann im Rahmen der Erfindung die Verwendung von Kombinationen von Lösungen verschiedener Antibiotika als Flüssigkeit vorgesehen sein. Beispielhaft sind hierbei die Zweifachkombinationen von Gentamcinsulfat mit Vancomycinhydrochlorid, die Zweifachkombination von Daptomycin mit Gentamicinsulfat und die Zweifachkombination von Gentamicinsulfat mit Clindamycin sowie die Dreifachkombination Gentamicinsulfat mit Vancomycinhydrochlorid und Clindamycinhydrochlorid.

Des Weiteren kann vorgesehen sein, dass als Flüssigkeit Antiseptika-Lösungen verwendet werden, insbesondere Lösungen des Chlorhexidindigluconats, des Octenidindihydrochlorids und des Polyhexanids.

Erfindungsgemäß kann auch vorgesehen sein, dass die Flüssigkeit Lösungen von Antibiotika und Antiseptika umfasst, die als Lösungsmittel organische Lösungsmittel oder Kombinationen von organischen Lösungsmitteln oder auch Kombinationen von organischen Lösungsmitteln und Wasser enthalten. Es ist dadurch möglich, zum Beispiel auch in Wasser gering lösliche Antibiotika-Salze, wie Laurate, Myristate, Palmitate und Stearate zu verwenden. Daneben können auch in Wasser gering lösliche Antibiotika oder Antibiotika-Salze in Form von wässrigen Suspensionen eingesetzt werden.

Eine besonders vorteilhafte Ausgestaltung eines erfindungsgemäßen Verfahrens sieht vor, dass die Flüssigkeit aus dem Übertragungsmittel heraus auf die zu beschichtende Oberfläche des medizinischen Implantats übertragen wird.

Die Poren des Übertragungsmittels können erfindungsgemäß durch Kanäle, Vorratskammern, Faserzwischenräume und/oder anderen Zwischenräumen des Übertragungsmittels realisiert werden.

Ferner ist es von besonderem Vorteil, wenn vorgesehen ist, dass die Flüssigkeit in das Übertragungsmittel eingebracht wird, vorzugsweise in Poren, Kanäle, Vorratskammern und/oder Zwischenräume des Übertragungsmittels, oder das Übertragungsmittel mit der Flüssigkeit getränkt wird, insbesondere unmittelbar vor der Verwendung. Dadurch wird die Variabilität des Verfahrens sichergestellt.

Dabei kann vorgesehen sein, dass eine zur Behandlungssituation passende Flüssigkeit, insbesondere ein zur Behandlungssituation passendes Antibiotikum oder Antibiotika-Gemisch in das Übertragungsmittel eingebracht wird. Eine an die speziellen Erfordernisse eines bestimmten Patienten angepasste Lösung kann somit kurz vor dem eigentlichen Beschichten des medizinischen Implantats bereitgestellt werden.

Gemäß einer besonders vorteilhaften und bevorzugten Ausgestaltung des erfindungsgemäßen Verfahrens kann vorgesehen sein, dass das Volumen des Übertragungsmittels durch Entspannen und/oder Dekomprimieren des Übertragungsmittels vergrößert wird.

Dabei wird die Rückstellkraft des elastischen Übertragungsmittels genutzt, um das Volumen des Übertragungsmittels zu vergrößern. Bei der Vergrößerung des Volumens des Übertragungsmittels öffnen sich die Poren des Übertragungsmittels. Die sich öffnenden Poren ziehen die Flüssigkeit ein, so dass sich das Übertragungsmittel mit der Flüssigkeit vollsaugt.

Gemäß einer weiteren Ausgestaltung der Erfindung kann vorgesehen sein, dass das Übertragungsmittel durch eine Komprimierungseinrichtung in dem komprimierten Zustand gehalten wird, wobei durch eine Bedienung der Vorrichtung, insbesondere der Komprimierungseinrichtung, das Volumen des Übertragungsmittels durch die Komprimierungseinrichtung vergrößert wird.

Dabei kann vorgesehen sein, dass als Komprimierungseinrichtung ein Deckel mit einem Gewinde verwendet wird, wobei das Gewinde in ein Gegengewinde der Vorrichtung greift, und dass durch Aufschrauben des Deckels das Volumen des Übertragungsmittels vergrößert wird.

Durch diese Maßnehmen wird ein einfacher, kostengünstig zu realisierender und leicht bedienbarer Aufbau geschaffen, der unter den schwierigen Bedingungen während einer OP einfach und sicher bedienbar ist.

Es kann ferner vorgesehen sein, dass die Flüssigkeit durch zumindest eine Durchführung in der Vorrichtung, insbesondere im Deckel der Vorrichtung eingefüllt wird, insbesondere unmittelbar vor der Übertragung auf das medizinische Implantat, wobei vorzugsweise die zuvor geschlossene Durchführung zuvor geöffnet wird, besonders bevorzugt indem eine Verschlussmembran durchstoßen wird.

Hierdurch wird das Verfahren variabel mit verschiedenen Flüssigkeiten anwendbar.

Eine weitere Ausgestaltung der Erfindung kann vorsehen, dass die Flüssigkeit vom Übertragungsmittel auf eine zu beschichtende Oberfläche des medizinischen Implantats übertragen wird, indem das medizinische Implantat durch einen Schlitz oder mehrere Schlitze und/oder eine Ausnehmung im Übertragungsmittel geschoben und wieder herausgezogen wird, wobei vorzugsweise beim Herausziehen überschüssige Flüssigkeit abgestreift wird.

Dabei kann vorgesehen sein, dass das Übertragungsmittel beim Hindurchschieben des medizinischen Implantats zumindest bereichsweise komprimiert wird und dadurch die Flüssigkeit zumindest teilweise aus dem Übertragungsmittel herausgedrückt wird und auf die Oberfläche des medizinischen Implantats übertragen wird, insbesondere allseitig auf die Oberfläche des medizinischen Implantats übertragen wird.

Bei diesen einfachen und leicht anwendbaren Verfahrensschritten wird wieder die Elastizität des Übertragungsmittels genutzt sowie die Tatsache, dass sich das Volumen der

Poren des Übertragungsmittels beim Komprimieren, also bei Krafteinwirkung verringert und die in den Poren enthaltene Flüssigkeit aus den Poren herausgedrückt wird, zur Oberfläche des Übertragungsmittels gelangt und dort auf die zu beschichtende Oberfläche des medizinischen Implantats übertragen wird.

Es kann auch vorgesehen sein, dass ein Antibiotikum oder Antibiotika-Gemisch als Flüssigkeit verwendet wird, vorzugsweise eine Suspension oder eine Lösung, besonders bevorzugt eine wässrige Lösung.

Ferner kann vorgesehen sein, dass nach dem Übertragen der Flüssigkeit auf das medizinische Implantat ein Pulver auf die benetzte Oberfläche des medizinischen Implantats aufgebracht wird, bevorzugt das medizinische Implantat in ein Pulver eingetaucht wird, wobei das Pulver zumindest eine pharmazeutisch aktive Substanz umfasst. Das Pulver kann in einem Hohlraum des Behälters unter dem Übertragungsmittel enthalten sein.

Eine zusätzliche Pulverbeschichtung ist für einige medizinische Anwendungen vorteilhaft, da hierdurch andere, nicht oder nur schwer lösbare medizinisch wirksame Substanzen oder Substanzen in anderen Konzentrationen oder Kombinationen einsetzbar sind. Durch die zuvor erfolgte Beschichtung mit einem Flüssigkeitsfilm, haftet das Pulver besonders gut auf der Oberfläche des medizinischen Implantats.

Eine weitere besonders bevorzugte Ausgestaltung der Erfindung kann vorsehen, dass eine eingefärbte Flüssigkeit verwendet wird, so dass der beschichtete Bereich des medizinischen Implantats farblich kenntlich gemacht wird, wobei vorzugsweise die Vollständigkeit der Beschichtung des zu beschichtenden Bereichs anhand der Färbung geprüft wird.

Hierdurch kann erfindungsgemäß sichergestellt werden, dass alle Bereiche des Implantats auch wirklich beschichtet wurden.

Besonders vorteilhafte Verfahren zeichnen sich dadurch aus, dass die Flüssigkeit mit einem ringförmigen Übertragungsmittel allseitig auf die zu beschichtende Oberfläche des medizinischen Implantats aufgebracht wird. Dies trägt der speziellen Geometrie vieler medizinischer Implantate, insbesondere von Gelenkprothesen Rechnung.

Zur Verhinderung einer Verunreinigung der Umgebung mit der Flüssigkeit kann vorgesehen sein, dass das medizinische Implantat vor dem Kontakt mit dem Übertragungsmittel in einen Behälter eingeführt wird, in dem sich das Übertragungsmittel befindet und nach der Übertragung der Flüssigkeit auf das medizinische Implantat aus dem Behälter herausgezogen wird.

Eine weitere Verbesserung kann dadurch erreicht werden, dass nach dem Übertragen der Flüssigkeit auf das medizinische Implantat ein Pulver auf die benetzte Oberfläche des medizinischen Implantats aufgebracht wird, bevorzugt das medizinische Implantat in ein Pulver eingetaucht wird, wobei das Pulver zumindest eine pharmazeutisch aktive Substanz umfasst. Ein geeignetes Pulver kann beispielsweise das Knochenwachstum fördern und so den Erfolg einer Implantation verbessern, da das Pulver eine stabilere Verbindung des Implantats mit dem angrenzenden Knochenmaterial bewirkt.

Auch kann vorgesehen sein, dass das medizinische Implantat durch zumindest eine Membran gestoßen wird oder zumindest eine Membran geöffnet wird. Dabei kann die zumindest eine Membran die Flüssigkeit und/oder das Pulver zumindest bereichsweise abdecken und/oder die zumindest eine Membran den Behälter abdichten. Die Membran verhindert eine Kontamination des Inneren des Behälters, der Flüssigkeit und/oder des Pulvers vor der Anwendung. Durch das Durchstoßen der Membran wird sichergestellt, dass die schützende Membran erst kurz vor der Anwendung geöffnet wird. Die Membran sollte dazu derart aufgebaut sein, dass keine Fetzen oder andere Teile der Membran auf das Übertragungsmittel beziehungsweise ins Innere des Behälters gelangen können oder am medizinischen Implantat haften.

Eine Reduzierung der Gefahr einer möglichen Verunreinigung der Umgebung und eine Einsparung der oft teuren Materialien zur Beschichtung lässt sich erfindungsgemäß dadurch erreichen, dass ein Teil der übertragenen Flüssigkeit abgestreift wird, insbesondere beim Herausziehen des medizinischen Implantats aus dem Behälter, vorzugsweise an einem dafür vorgesehenen Abstreifer. Hierdurch kann eine Kontamination der Umgebung, also insbesondere eines OP-Bereichs mit der Flüssigkeit und gegebenenfalls auch mit dem Pulver verhindert oder zumindest reduziert werden. Dies ist vor allem bei der Verwendung von Antibiotika geboten, da so der Entwicklung resistenter Keime im OP-Bereich vorgebeugt werden kann.

Erfindungsgemäß kann auch vorgesehen sein, dass das zu beschichtende medizinische Implantat mehrfach in die Vorrichtung eingesteckt wird beziehungsweise mehrfach durch das Übertragungsmittel gesteckt wird.

Es kann ferner vorgesehen sein, dass das Verfahren so oft wiederholt wird, bis eine vollständige Beschichtung der zu beschichtenden Oberfläche des medizinischen Implantats erreicht wird. Insbesondere im Zusammenhang mit einer Färbung der Flüssigkeit und der Prüfung der Vollständigkeit der Beschichtung mit Hilfe der Färbung ist dies erfindungsgemäß vorteilhaft, um eine ausreichend beschichtetes medizinisches Implantat zu erhalten.

Auch kann erfindungsgemäß vorgesehen sein, dass zumindest 50% der Oberfläche des medizinischen Implantats, vorzugsweise zumindest 80%, besonders bevorzugt 90% der Oberfläche des medizinischen Implantats beschichtet werden.

Eine besonders vorteilhafte Ausgestaltung des Verfahrens ist dadurch gekennzeichnet, dass als medizinisches Implantat nicht zu zementierende Hüftgelenkendoprothesen, Schultergelenkendoprothesen, Ellenbogenprothesen, Marknägel oder Osteosyntheseplatten verwendet werden.

Die Aufgaben der Erfindung sowie weitere nicht genannte Probleme werden auch gelöst durch eine Vorrichtung zur zumindest bereichsweisen Beschichtung eines medizinischen Implantats, insbesondere mit einem solchen Verfahren, wobei die Vorrichtung zumindest ein zumindest bereichsweise komprimiertes, elastisches, poröses Übertragungsmittel umfasst.

Die Vorrichtung ist besonders bevorzugt eine medizinische Beschichtungsvorrichtung für medizinische Implantate. Immer wenn erfindungsgemäß von einer Vorrichtung die Rede ist, kann erfindungsgemäß eine solche medizinische Beschichtungsvorrichtung für medizinische Implantate gemeint sein.

Dabei kann vorgesehen sein, dass die Vorrichtung eine Komprimierungseinrichtung umfasst, wobei das Übertragungsmittel durch die Komprimierungseinrichtung in einem komprimierten Zustand haltbar ist und vorzugsweise durch Bedienen der Vorrichtung, insbesondere der Komprimierungseinrichtung das Volumen des Übertragungsmittels vergrößerbar ist.

Dabei kann wiederum vorgesehen sein, dass die Komprimierungseinrichtung ein Deckel mit einem Gewinde ist, der in die Vorrichtung eingeschraubt ist, wobei das Volumen des Übertragungsmittels durch Drehen und/oder Schrauben des Deckels vergrößerbar ist.

Hierbei kann es vorteilhaft sein, wenn vorgesehen ist, dass der Deckel eine Durchführung umfasst, durch die eine Flüssigkeit ins Innere der Vorrichtung im Bereich des Übertragungsmittels einbringbar ist, wobei die Durchführung vorzugsweise in eine Verteilvorrichtung mündet, mit der die Flüssigkeit zumindest bereichsweise auf der Oberfläche des Übertragungsmittels verteilbar ist und die Verteilvorrichtung besonders bevorzugt radial angeordnete Rippen umfasst.

Gemäß einer weiteren Ausgestaltung der Erfindung kann vorgesehen sein, dass zwischen der Komprimierungseinrichtung und dem Übertragungsmittel ein beweglicher Haltering angeordnet ist, der das zumindest eine Übertragungsmittel im Inneren der Vorrichtung hält, insbesondere unabhängig von der Komprimierung des Übertragungsmittels, wobei der Haltering vorzugsweise eine Arretierungsvorrichtung umfasst, die mit einem Anschlag der Vorrichtung den eingesetzten Haltering und damit das Übertragungsmittel in der Vorrichtung hält, besonders bevorzugt die Bewegung des Halterings in der Vorrichtung begrenzt.

Eine weitere Ausgestaltung der Erfindung kann vorsehen, dass das Übertragungsmittel in einem Behälter umfassend eine Öffnung zum Einführen und Herausnehmen des medizinischen Implantats angeordnet ist, wobei die Öffnung vorzugsweise durch den Deckel geschlossen ist.

Eine erfindungsgemäß besonders bevorzugte Ausgestaltung der Erfindung kann dadurch realisiert werden, dass die Vorrichtung einen Abstreifer umfasst, der vorzugsweise im Bereich der Öffnung, insbesondere zwischen der Öffnung und dem Übertragungsmittel angeordnet ist.

Mit dem Abstreifer kann überschüssige Flüssigkeit vom medizinischen Implantat beim Herausziehen des medizinischen Implantats aus der Vorrichtung entfernt werden. Dabei kann vorgesehen sein, dass der Abstreifer scheibenförmig ist und mindestens einen Einschnitt umfasst, der die Oberseite und die Unterseite der Scheibe verbindet.

Alternativ kann vorgesehen sein, dass der Abstreifer als Kegelmantel oder als Halbkugelfläche ausgebildet ist, wobei die Kegelspitze oder die Halbkugel in Richtung des Übertragungsmittels ausgerichtet ist und vorzugsweise der Kegel oder die Halbkugel mindestens einen Einschnitt enthalten, der die Oberseite und die Unterseite des Abstreifers verbindet. Dadurch können sich eventuell vom Übertragungsmittel während des Herausziehens des Implantats ablösende Tröpfchen und Partikel nur so umhergeschleudert werden, dass diese gegen die Innenwand des Behälters und gegen die Unterseite des Abstreifers prallen und somit eine Kontamination der Umgebung mit der Flüssigkeit vermindert wird.

Es kann ferner vorgesehen sein, dass das Übertragungsmittel mindestens einen Einschnitt umfasst, vorzugsweise mehrere Übertragungsmittel jeweils zumindest einen Einschnitt umfassen, wobei die Übertragungsmittel derart zueinander angeordnet sind, dass die Einschnitte zueinander versetzt angeordnet sind.

Hierdurch wird die allseitige Beschichtung des medizinischen Implantats unterstützt beziehungsweise sichergestellt.

Eine weitere Ausgestaltung der Erfindung kann vorsehen, dass das Übertragungsmittel ein Schwamm ist oder die Übertragungsmittel Schwämme sind, mit dem oder mit denen die Flüssigkeit auf die zu beschichtende Oberfläche des medizinischen Implantats übertragbar ist, wobei der Schwamm oder die Schwämme vorzugsweise aus einem Material mit hydrophiler Oberfläche gefertigt ist oder sind, wobei besonders bevorzugt zumindest ein anderes Teil der Vorrichtung, insbesondere der Abstreifer aus einem hydrophoben Material gefertigt sind.

Ferner kann vorgesehen sein, dass unterhalb des Übertragungsmittels ein Hohlraum angeordnet ist, wobei das Übertragungsmittel vorzugsweise auf einer durchstoßbaren Membran angeordnet ist, die das Innere des Hohlraums abschließt.

Es kann vorgesehen sein, dass das Übertragungsmittel in einem Behälter umfassend eine Öffnung zum Einführen und Herausnehmen des medizinischen Implantats angeordnet ist. Der Behälter dient dazu, ein ungewolltes Verspritzen und Abtropfen der Flüssigkeit in die Umgebung zu vermeiden.

Dabei kann wiederum vorgesehen sein, dass die Öffnung durch einen Deckel verschlossen ist. Dadurch können Verunreinigungen des Inhalts vor der Anwendung der Vorrichtung vermieden werden.

Besonders vorteilhafte Vorrichtungen zeichnen sich dadurch aus, dass das Übertragungsmittel Poren umfasst und die Flüssigkeit in die Poren des Übertragungsmittels die Flüssigkeit, vorzugsweise in Form einer Lösung und/oder Suspension aufnehmbar ist und/oder enthalten ist.

Eine weitere Ausgestaltung der Erfindung sieht vor, dass die pharmazeutisch aktive Substanz Antibiotika und/oder organische Antiseptika beinhaltet, so dass die zu erzeugende Beschichtung eine pharmazeutisch wirksame Dosis beinhaltet.

Auch kann vorgesehen sein, dass die Vorrichtung einen Vakuumanschluss umfasst, der mit einer Vakuumquelle verbindbar ist und der vorzugsweise zwischen dem Abstreifer und dem Übertragungsmittel angeordnet ist. Dadurch kann zusätzlich durch Absaugen von eventuellen Tröpfchen der Lösung und/oder Suspension sichergestellt werden, dass keine Kontamination der Umgebung mit pharmazeutischen Wirkstoffen erfolgt.

Gemäß einer Ausgestaltung der Erfindung kann vorgesehen sein, dass das Übertragungsmittel aus einem hydrophilen Material gefertigt ist und vorzugsweise zumindest ein anderes Teil, insbesondere der Behälter und/oder der Abstreifer aus einem hydrophoben Material gefertigt sind. Bevorzugt werden wässrige Lösungen und/oder Suspensionen von pharmazeutischen Wirkstoffen für die Beschichtung verwendet. Wenn das Übertragungsmittel aus einem hydrophilen Material gefertigt ist, befinden sich wässrige Lösungen und/oder Suspensionen bevorzugt in dem porösen hydrophilen Material und nicht auf der hydrophoben Oberfläche des Behälters und des Abstreifers. Dieses Verhalten ermöglicht es, das mit wässrigen Lösungen und/oder Suspensionen vorgefüllte Beschichtungsvorrichtungen mit geringsten Volumina dieser wässrigen Lösungen oder Suspensionen auskommen und trotzdem eine sichere Beschichtung ermöglichen.

Ferner kann vorgesehen sein, dass der Abstreifer aus biokompatiblem Elastomer, thermoplastischem Kunststoff, aus einer Metallfolie oder aus Kompositen, die aus Metall-Elastomer-Kombinationen oder Metall-Kunststoffkombinationen gefertigt sind, aufgebaut ist.

Eine weitere Ausgestaltung der Erfindung sieht vor, dass der Abstreifer als Ring ausgebildet ist, der radial zum Mittelpunkt des Behälters angeordnete Borsten enthält, die gegebenenfalls einen Kegel bilden können, dessen Spitze in Richtung des Übertragungsmittels angeordnet ist. Diese Borsten können aus Kunststoff gebildet sein, wobei die Borsten mechanisch so stabil sein sollten und so stabil verankert sein sollten, dass eine Ablösung oder ein Abbrechen der Borsten möglichst unterbleibt.

Auch kann vorgesehen sein, dass der Abstreifer als durch Walzen und/oder Kugeln ausgebildet ist, die durch elastische Verbindungsmittel mit dem Behälter verbunden sind.

Ferner kann vorgesehen sein, dass mindestens ein Einschnitt die Oberseite des Übertragungsmittels mit der Unterseite des Übertragungsmittels verbindet. Durch diesen mindesten einen Einschnitt kann das medizinische Implantat durch das Übertragungsmittel hindurch gesteckt beziehungsweise geschoben werden.

Besonders vorteilhaft ist es, wenn dabei vorgesehen ist, dass radiale Einschnitte in dem Übertragungsmittel ausgebildet sind.

Der Erfindung liegt die überraschende Erkenntnis zugrunde, dass sich eine Flüssigkeit, mit der ein medizinisches Implantat vor einer OP beschichtet werden soll, leicht, schnell und ohne Kontamination der OP-Umgebung von einem sich dekomprimierenden Übertragungsmittel aufgesaugt werden kann. Das Befüllen des Übertragungsmittels mit der Flüssigkeit geschieht also, während sich das Übertragungsmittel ausdehnt und in Kontakt mit der Flüssigkeit befindet. Mit dem mit der Flüssigkeit vollgesogenen Übertragungsmittel lässt sich die Flüssigkeit dann leicht auf die Oberfläche des medizinischen Implantats übertragen. Die Flüssigkeit kann unmittelbar vor der Anwendung in die Vorrichtung gefüllt werden. So wird durch diese Anwendung eine Beschichtung ermöglicht, die während einer OP oder kurz zuvor aufgetragen wird. Dadurch lassen sich die Beschichtungen besser an die individuellen Bedürfnisse der Patienten anpassen und es können auch pharmazeutisch wirksame Substanzen mit kürzeren Halbwertszeiten eingesetzt werden. Gleichzeitig wird das Verspritzen der Flüssigkeit vermindert und so eine Kontamination der Umgebung verhindert. Dies ist vor allem bei einer Anwendung in einem Operationssaal wichtig.

Das schnelle und effiziente Befüllen des Übertragungsmittels, bei dem es sich vorzugsweise um einen Schwamm handelt, ist durch die Entspannung beziehungsweise Ausdehnung des Übertragungsmittels möglich.

Damit wird ein erfindungsgemäßes Verfahren zur schnellen und unkomplizierten Beschichtung von medizinischen Implantaten mit pharmazeutischen Zubereitungen unter OP-Bedingungen bereitgestellt. Bei vielen erfindungsgemäßen Ausgestaltungen der Erfindung wird zudem erreicht, dass die Freisetzung von Wirkstofftröpfchen oder Wirkstoffspritzern in den OP-Bereich weitgehend vermieden wird. Die Beschichtungsvorrichtung ist besonders zur kostengünstigen Beschichtung von nicht-zementierten Gelenkendoprothesen, nicht-zementierten Revisions-Gelenkendoprothesen und von Osteosynthesematerialien mit pharmazeutischen Wirkstoffen vorgesehen.

Anstatt also das medizinische Implantat lange im Voraus bei der Herstellung zu beschichten, kann es auch unmittelbar vor dem Einsetzen mit überschaubaren Mengen einer pharmazeutisch aktiven Substanz beschichtet werden. Dadurch können auch relativ kurzlebige Beschichtungen verwendet werden. Zudem kann sogar eine noch flüssige Schicht verwendet werden, was neue Anwendungsgebiete eröffnet und neue Wirkstoffe zugänglich macht.

Die nicht befüllte Vorrichtung kann direkt im OP durch Einspritzen einer Wirkstofflösung oder Wirkstoffsuspension mit einem oder mehreren pharmazeutischen Wirkstoffen befüllt werden. Dadurch ist es im Fall der antibiotischen Beschichtung möglich, entsprechend der vorliegenden Resistenzsituation eine geeignete Auswahl eines Antibiotikums oder einer Antibiotika-Kombination vorzunehmen und damit eine Antibiogrammgerechte Beschichtung herzustellen.

Es ist auch möglich, vor einer OP nicht befüllte Vorrichtungen in der jeweiligen Klinikapotheke mit geeigneten Wirkstofflösungen oder Wirkstoffsuspensionen zu befüllen, so dass während der OP die Beschichtung ohne Zeitverzug vorgenommen werden kann.

Besonders geeignet ist dabei erfindungsgemäß, dass als pharmazeutisch aktive Substanz ein pharmazeutischer Wirkstoff wie beispielsweise Antibiotika, organische Antiseptika, Kupfersalze, Kupferoxid, Galliumsalze, Strontiumsalze, Lithiumsalze, Silbersalze, Silberoxid, Bisphosphonate, Wachstumsfaktoren, Steroidhormone, nichtsteroidale Hormone, Hämostyptika, Antiphlogistika, Plasmide, Cosmide, lineare DNA und deren Mischungen angewendet werden.

Für einen initialen antibiotischen Schutz reicht es aus, wenn für einen Zeitraum von 24 bis 72 Stunden hinreichend hohe Antibiotikum- oder Antibiotika-Konzentrationen an den lmplantatoberflächen vorhanden sind. Daher kann schon bei der lokalen Einbringung von einfachen in Wasser löslichen Antibiotika ein ausreichender temporärer, lokaler, antibiotischer Schutz des medizinischen Implantats erzielt werden.

Die Vorrichtung kann als Arzneimittel oder als Medizinprodukt bereitgestellt werden.

Auch eine Kombination der erfindungsgemäßen Vorrichtung mit einem medizinischen Implantat könnte angeboten werden. Diese Kombination wird aus der Vorrichtung und dem Implantat gebildet, wobei diese Kombination eine Mindestlebensdauer von 0,1 Sekunden hat. Die Kombination entsteht während des Beschichtungsprozesses.

Erfindungsgemäß ist auch eine medizinische Beschichtungsvorrichtung zur Beschichtung mit flüssigen pharmazeutischen Zubereitungen von medizinischen Implantaten, die einen Hohlkörper mit einem offenen und einem geschlossenen Ende und zumindest eines der nachfolgenden Merkmale umfasst:
A) mindestens ein elastisches, offen-poröses Übertragungsmittel, das am offenen Ende des Hohlkörpers angeordnet ist;
B) mindestens eine Auflage für das Übertragungsmittel an der Innenseite des Hohlkörpers;
C) mindestens eine Membran, welche die Unterseite des elastischen, offen-porösen Übertragungsmittels in Richtung der Innenseite des Hohlkörpers verschließt;
D) mindestens einen Führungsring, der das Übertragungsmittel in Richtung des offenen Endes des Hohlkörpers blockiert;
E) mindestens einen Abstreifer;
F) ein Innengewindegewinde oder zu mindestens ein Gewindeausschnitt eines Innengewindes am offenen Ende des Hohlkörpers; und/oder
G) einen Schraubdeckel, der mindestens ein Außengewinde umfasst, bei dem an der Unterseite Rippen oder Stege angeordnet sind, wobei die Flächen zwischen den Rippen oder Stegen über mindestens eine Öffnung verbunden sind, welche die Oberseite des Schraubdeckels mit der Unterseite verbindet.

Bei Merkmalskombination G) ist bevorzugt das Gesamtvolumen der Hohlräume zwischen den Rippen oder Stegen auf der Schraubdeckelunterseite und des auf der Rückseite des Schraubdeckels angeordneten Hohlkörpers kleiner oder gleich dem gesamten Porenvolumen des elastischen, offen-porösen Übertragungsmittels.

Vorzugsweise ist bei Merkmalskombination G) die Öffnung an der Schraubdeckeloberseite als Hohlkörper, insbesondere als Rohr ausgebildet.

Die Erfindung basiert auf der Idee, ein elastisches, offen-poröses Übertragungsmittel zu verwenden, das bei Verformung eine hohe Rückstellkraft hat. Wenn ein elastisches, offen-poröses Material zusammengepresst wird, dann entweicht die in den Poren enthaltene Luft. Bei Nachlassen der Kompression kehrt das Material auf Grund seiner Rückstellkraft in seine Ausgangsvolumen zurück, wobei die sich wieder öffnenden Poren umgebendes gasförmiges oder flüssiges Medium aufnehmen.

Im Folgenden werden Ausführungsbeispiele der Erfindung anhand von sechs schematisch dargestellten Figuren erläutert, ohne jedoch dabei die Erfindung zu beschränken. Dabei zeigt:
- Figur 1:: eine schematische Querschnittansicht einer erfindungsgemäßen Vorrichtung mit einem komprimierten Übertragungsmittel;
- Figur 2:: eine schematische Querschnittansicht der erfindungsgemäßen Vorrichtung mit einem entspannten Übertragungsmittel;
- Figur 3:: eine schematische perspektivische Ansicht eines Deckels als Komprimierungseinrichtung für eine erfindungsgemäße Vorrichtung;
- Figur 4:: eine schematische Aufsicht auf die Oberseite eines Deckels als Komprimierungseinrichtung für eine erfindungsgemäße Vorrichtung;
- Figur 5:: eine schematische Aufsicht auf die Unterseite eines Deckels als Komprimierungseinrichtung für eine erfindungsgemäße Vorrichtung; und
- Figur 6:: eine schematische perspektivische Querschnittansicht eines Halterings für eine erfindungsgemäße Vorrichtung.

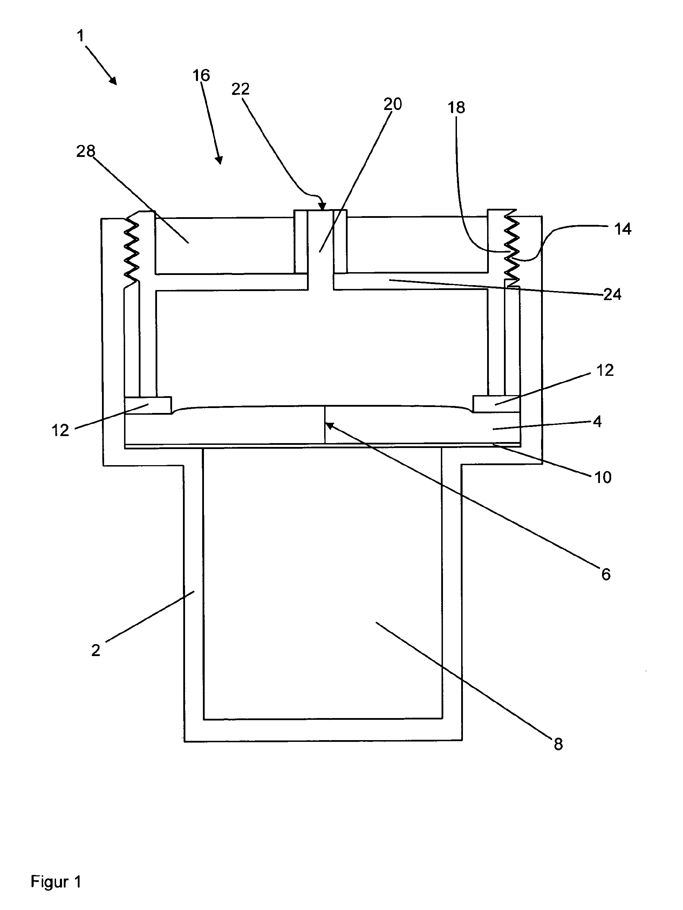

Figur 1 zeigt eine schematische Querschnittansicht einer erfindungsgemäßen Vorrichtung 1. Die Vorrichtung 1 umfasst einen nach oben geöffneten Behälter 2. Die Seitenwände des Behälters 2 sind zylindrisch und von gleichmäßiger Dicke. Der Boden und die Seitenwände des Behälters 2 sind aus einem hydrophoben Material gefertigt oder mit einer hydrophoben Schicht beschichtet.

Im Inneren des Behälters 2 ist ein elastisches Übertragungsmittel 4 angeordnet, das beispielsweise durch einen Schwamm realisiert werden kann. Ausgehend von einer Öffnung 6 des Übertragungsmittels 4 ist das Übertragungsmittel 4 in acht Richtungen geschlitzt beziehungsweise eingeschnitten.

Die acht Schlitze / Einschnitte 6 reichen nicht bis zu den Seitenwänden des Behälters 2 und sollen das Durchstecken eines medizinischen Implantats durch das Übertragungsmittel 4 ermöglichen. Das Übertragungsmittel 4 hat dadurch acht flexible Segmente die beim Einführen und beim Herausnehmen, beziehungsweise beim Herausziehen des medizinischen Implantats dessen Oberfläche überstreichen. Die Öffnung 6 kann auch eine kreisrunde Ausnehmung sein, deren Durchmesser kleiner als der Querschnitt der einzuführenden medizinischen Implantate gewählt wird. Dadurch ist sichergestellt, dass das Übertragungsmittel 4 die gesamte Oberfläche des medizinischen Implantats überstreicht und dabei beschichtet.

Das Übertragungsmittel 4 ist aus einem flexiblen, porösen Material gefertigt. Das Material ist hydrophil. Dadurch wird sichergestellt, dass es leicht mit einer wässrigen Flüssigkeit getränkt werden kann. Durch die hydrophoben Eigenschaften des Behälters 2 wird eine wässrige Flüssigkeit, die in die Vorrichtung 1 eingefüllt wird, vor allem vom Übertragungsmittel 4 aufgenommen. Das Übertragungsmittel 4 wird sich also mit einer wässrigen Lösung umfassend ein Antibiotikum leicht vollsaugen können.

Der Behälter 2 bildet am unteren Ende der Vorrichtung 1 einen Hohlraum 8, der vollständig geschlossen ist und auf der Oberseite durch eine Membran 10 abgeschlossen ist. Auf der Membran 10 ist das Übertragungsmittel 4 angeordnet. Auf der Oberseite des Übertragungsmittels 4 ist ein Haltering 12 angeordnet der entlang der Längsasche der Vorrichtung 1 beweglich ist. Das Übertragungsmittel 4 lässt sich als zwischen der Membran 10 und dem Haltering 12 zusammenpressen beziehungsweise komprimieren.

Am oberen Ende des Behälters 2 hat dieser eine Öffnung. Im Bereich der Öffnung ist an der Oberseite des Behälters 2 ein Innengewinde 14 angeordnet. Der Haltering 12 kann im Behälter 2 nicht in den Bereich des Innengewindes 14 vordringen, da dieses übersteht und damit dem Haltering den Weg versperrt. Der Haltering 12 ist flach und circa 1 mm dick oder dünner, vorzugsweise 0,5 mm bis 1 mm dick. Der Haltering 12 dient dazu, das Übertragungsmittel 4 im Inneren des Behälters 2 zu halten. Am Haltering 12 kann eine Rastvorrichtung (nicht gezeigt) angeordnet sein, so dass der Haltering 12 problemlos in den Behälter 2 eingesetzt werden kann, nicht aber wieder herausfallen kann.

Eine Abstufung des Behälters 2 trennt den Bereich für das Übertragungsmittel 4 und den Haltering 12 vom Hohlraum 8. Die Öffnung des Behälters 2 ist durch einen Deckel 16 verschlossen, der auch als Komprimierungseinrichtung für das Übertragungsmittel 4 dient. Der Deckel 16 ist außen zylindrisch geformt und umfasst ein Außengewinde 18, das in das Innengewinde 14 des Behälters 2 greift. In Figur 1 ist der Deckel 16 tief in den Behälter 2 geschraubt. Dadurch wird der Haltering 12 nach unten in Richtung der Abstufung des Behälters 2 gedrückt. Dazu wird das poröse, elastische Übertragungsmittel 4 und damit die im Übertragungsmittel 4 enthaltenen Poren zusammengepresst.

Durch eine rohrförmige Durchführung 20 im Deckel 16 kann eine Flüssigkeit in den Behälter 2 eingefüllt werden. Die Durchführung 20 ist vorliegend durch eine Membran 22 abgedeckt, die entweder abgezogen oder durchstochen werden kann, um die Vorrichtung 1 mit einer Flüssigkeit zu befüllen.

Der Deckel 16 ist durch eine Trennwand 24 zweigeteilt. Der untere Bereich, in den die Durchführung 20 mündet, bildet einen Hohlraum im Deckel 16 beziehungsweise der Vorrichtung 1, in dem Rippen (nicht gezeigt) angeordnet sind, die für eine Verteilung einer eingefüllten Flüssigkeit in diesem Hohlraum sorgen und auch gleichzeitig auf das Übertragungsmittel 4 drücken können und so auch zur Komprimierung des Übertragungsmittels 4 beitragen können. Die Unterseite des Deckels 16 könnte auch durch eine perforierte Platte begrenzt sein, dabei ist wichtig, dass eine in den Hohlraum im Deckel 16 eingefüllte Flüssigkeit auf eine möglichst große Oberfläche des Übertragungsmittels 4 geleitet wird und die Strömung zu dieser Oberfläche nicht unnötig behindert wird.

Auf der Oberseite des Deckels 16 sind zwei Flügel 28 angeordnet, mit denen der Deckel 16 leicht manuell geschraubt werden kann.

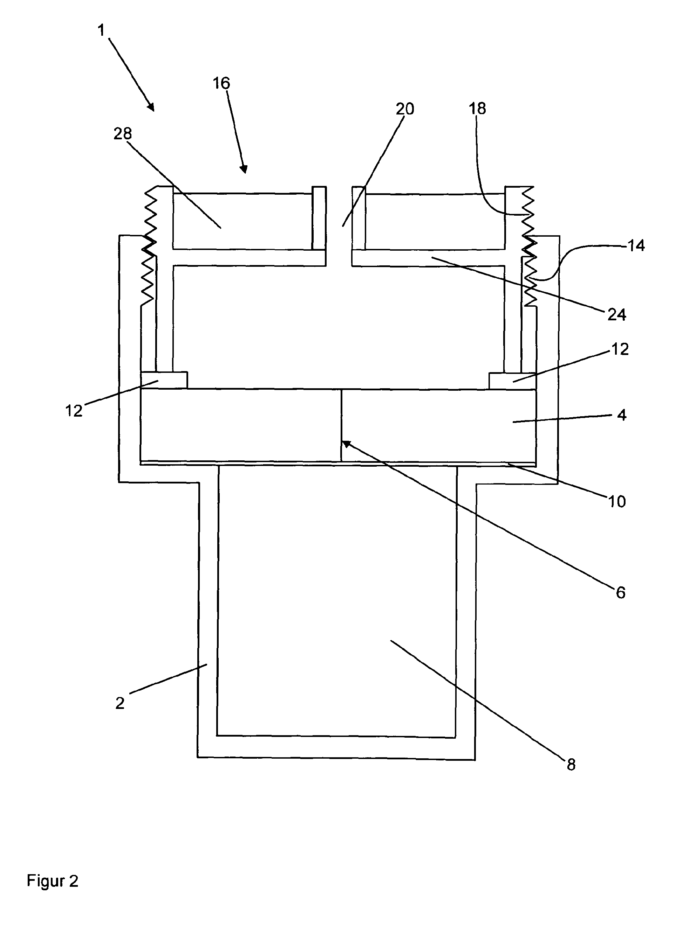

Mit der gezeigten Vorrichtung 1 kann ein erfindungsgemäßes Verfahren durchgeführt werden. Die Membran 22 auf der Durchführung 20 wird durchstochen oder entfernt. Anschließend wird eine wässrige Flüssigkeit umfassend zumindest eine pharmazeutisch wirksame Substanz, vorzugsweise eine Beschichtungslösung oder eine Beschichtungssuspension, durch die Durchführung 20 in den Hohlraum im Deckel 16 und damit in den Behälter 2 und die Vorrichtung 1 eingefüllt. Dies kann beispielsweise mit einer Spritze geschehen. Die Flüssigkeit kann nicht in den durch die Membran 10 verschlossenen Hohlraum 8 eindringen. Anschließend wird der Deckel 16 aus dem Behälter 2 heraus geschraubt. Dabei dehnt sich das Übertragungsmittel 4 während des Herausschraubens aus. Der Zustand mit weitgehend herausgeschraubtem Deckel 16 ist in Figur 2 gezeigt.

Durch die Ausdehnung des Übertragungsmittels 4 öffnen sich auch wieder die Poren in dem Übertragungsmittel 4. Beim Öffnen der Poren saugen sich diese mit der Flüssigkeit voll. Die elastische Federkraft des Übertragungsmittels 4 wird also genutzt, um eine Druckdifferenz zwischen den Hohlräumen/Poren im Übertragungsmittel 4 und dem Umgebungsdruck zu erzeugen, so dass die Flüssigkeit in die Hohlräume, also die Poren des Übertragungsmittels 4 hereingedrückt beziehungsweise hineingesaugt wird. Die Vorrichtung 1 sollte dazu in etwa senkrecht gehalten oder gelagert sein, damit die Flüssigkeit in dem Hohlraum des Deckels 16 auf der Oberfläche des Übertragungsmittels 4 lastet. Dazu kann an der Unterseite der Vorrichtung 1 ein Ständer (nicht gezeigt) angeordnet sein.

Derart wird das Übertragungsmittel 4 mit der wässrigen Flüssigkeit getränkt, die zumindest eine pharmazeutisch wirksame Substanz enthält, mit der ein medizinisches Implantat beschichtet werden soll. Der Deckel 16 kann kurz vor der Verwendung der Vorrichtung 1 zum Beschichten des medizinischen Implantats entfernt werden.

Ein medizinisches Implantat (nicht gezeigt) wird durch die Öffnung des Behälters 2 hindurch geschoben und trifft auf das Übertragungsmittel 4. Durch den Druck, der über das medizinische Implantat auf das Übertragungsmittel 4 ausgeübt wird schiebt sich zum einen das medizinische Implantat durch die dafür vorgesehenen Durchbrechung 6 im Übertragungsmittel 4 und zum anderen wird die in den Poren des Übertragungsmittels 4 enthaltene Flüssigkeit aus dem Übertragungsmittel 4 herausgedrückt und auf die Oberfläche des medizinischen Implantats übertragen. Das medizinische Implantat durchstößt die Membran 10 und dringt in den Hohlraum 8 ein. Der Hohlraum 8 nimmt herabtropfende Flüssigkeitstropfen auf und ist groß genug, um die durch das Übertragungsmittel 4 geschobenen Teile des medizinischen Implantats aufzunehmen. In dem Hohlraum 8 kann auch ein Pulver umfassend eine pharmazeutisch wirksame Substanz enthalten sein, in das das mit der Flüssigkeit benetzte medizinische Implantat eintaucht und dabei das Pulver zum Teil auf der Oberfläche des medizinischen Implantats haften bleibt.

Nachdem die Oberfläche des medizinischen Implantats beschichtet wurde, wird es aus dem Behälter 4 herausgezogen. Dabei kann die beschichtete Oberfläche des medizinischen Implantats an einem Abstreifer (nicht gezeigt) vorbeigezogen werden. Überschüssige Flüssigkeit wird durch den Abstreifer von der Oberfläche des medizinischen Implantats abgestreift und tropft zurück in den Behälter 2. Das aus dem Behälter 2 herausgezogene medizinische Implantat tropft dann nicht mehr nach. Durch diese Maßnahmen kann verhindert werden, dass die Flüssigkeit die Umgebung kontaminiert. Das mit der Flüssigkeit beschichtete medizinische Implantat ist dann bereit, um für eine Operation eingesetzt zu werden.

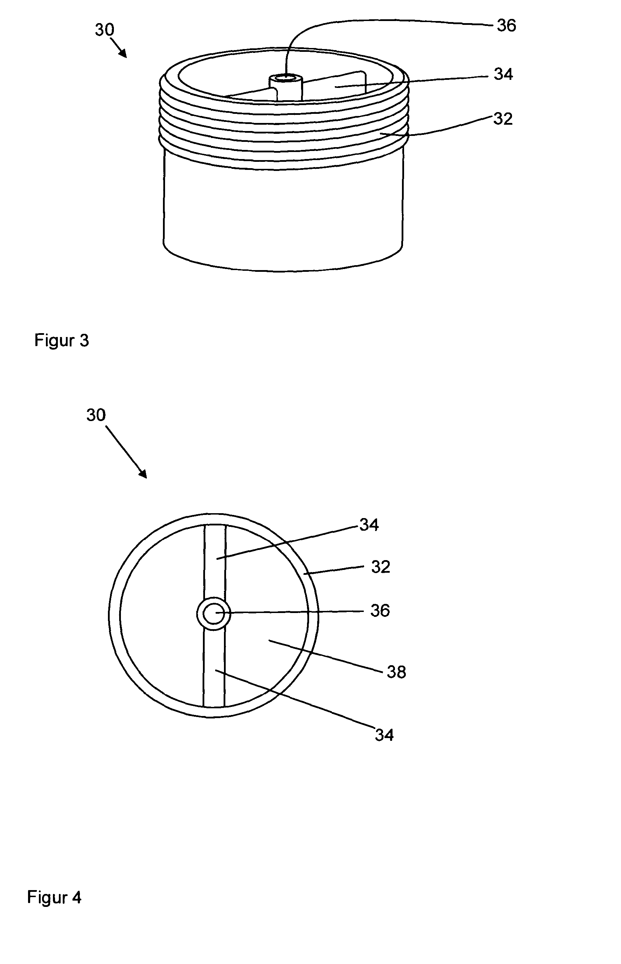

Figur 3 zeigt eine schematische perspektivische Ansicht einer Komprimierungsvorrichtung in Form eines schraubbaren Deckels 30 für eine erfindungsgemäße Vorrichtung. Der aus Kunststoff gefertigte Deckel 30 ist zylindrisch geformt und weist auf der Außenseite ein Außengewinde 32 auf. Zur besseren Handhabung sind im Deckel 30 zwei Flügel 34 angeordnet, so dass ein Anwender mit der Hand beziehungsweise den Fingern in den Deckel 30 eingreifen kann und den Deckel 30 manuell in die Vorrichtung (nicht gezeigt) hineinschrauben oder aus der Vorrichtung herausschrauben kann.

In der Mitte des Deckels 30 ist entlang der Symmetrieachse ein Rohr als Durchführung 36 angeordnet. Die Durchführung 36 reicht vorzugsweise nicht bis zur Unterseite des Deckels 30 sondern endet darüber. Im Bereich der Unterkante des Außengewindes 32 befindet sich im Inneren des Deckels 30 eine Trennwand (nicht zu sehen), die nur von der Durchführung 36 durchbrochen wird. Der darunter befindliche Raum ist zu Auffüllen mit der Flüssigkeit zur Beschichtung des medizinischen Implantats gedacht.

Figur 4 zeigt den Deckel 30 nach Figur 3 in einer schematischen Aufsicht von oben. Von oben ist die Trennwand 38 zu erkennen. Die zentrische Anordnung der Durchführung 36 im Deckel 30 ist ebenfalls gut zu sehen.

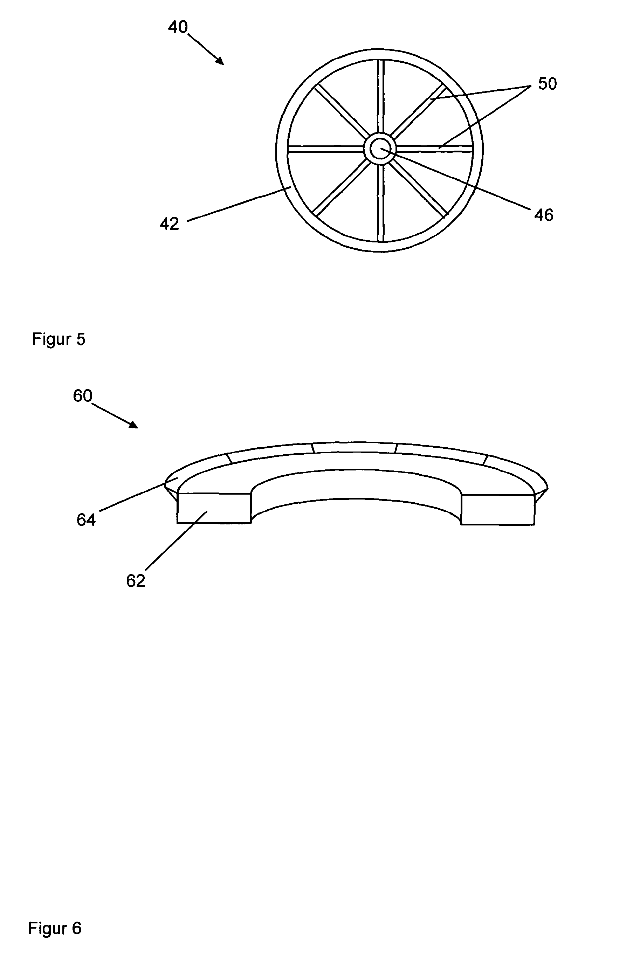

Figur 5 zeigt eine schematische perspektivische Aufsicht auf die Unterseite eines Deckels 40 als Komprimierungsvorrichtung. Die Wände 42 des Deckels 40 haben zylindrische Geometrie. Entlang der Symmetrieachse des Deckels 40 erstreckt sich im Bereich der Oberseite bis zu einer mittleren Höhe eine Durchführung 46, die eine Trennwand 48 durchbricht. Auf der Unterseite der Trennwand 48 ist der zur Bildung des Hohlraums im Deckel 40 vorgesehene Bereich durch Rippen 50 in acht Teile unterteilt. Die Rippen 50 dienen zur Verteilung der Flüssigkeit und zur Komprimierung des Übertragungsmittels (nicht gezeigt).

Figur 6 zeigt in perspektivischer schematischer Ansicht einen Haltering 60, der einen Ring 62 und eine segmentierte, bewegliche Haltevorrichtung 64 umfasst. Die Haltevorrichtung 64 soll sicherstellen, dass der Haltering 60, wenn er in eine medizinische Beschichtungsvorrichtung eingesetzt ist, nicht mehr ohne weiteres aus der Vorrichtung entfernt werden kann. Dazu kann die Haltevorrichtung 64 beispielsweise in den untersten Bereich des Innengewindes 14 für den Deckel 16 nach Figur 1 und Figur 2 greifen.

Anstatt der Verwendung des Deckels 16, 30, 40 als Komprimierungseinrichtung kann auch eine alternative Komprimierungseinrichtung zum Komprimieren des Übertragungsmittels 4 verwendet werden. Beispielsweise könnte eine Manschette in Form eines breiten Bands um ein Übertragungsmittel gelegt werden, wobei die Manschette zusammengezogen ist und so das Volumen des Übertragungsmittels verkleinert zusammenhält. Dazu könnte die Manschette eine Schraubvorrichtung umfassen, so dass der Umfang der Manschette und damit das Volumen langsam vergrößert werden kann. Die Manschette wäre dann nach Art einer Schlauchschelle aufgebaut. Ebenso könnte die Manschette aber auch eine lösbare Arretierung aufweisen, mit der die Manschette komplett lösbar beziehungsweise zu öffnen ist, so dass sich das Volumen des Übertragungsmittels sofort ausdehnt. In solchen Fällen ist es bevorzugt, wenn das Übertragungsmittel aus einen Material besteht, dessen Elastizität nur eine nicht sprunghafte Vergrößerung des Volumens erlaubt. Dadurch soll ein gleichmäßiges und kompletteres Befüllen der Poren des Übertragungsmittels mit der Flüssigkeit sichergestellt werden. Weitere Ausgestaltungen von Komprimierungseinrichtungen, beispielsweise auch durch den Behälter und dessen Geometrie sind vorstellbar.

Der Schraubdeckel 16, 30, 40 drückt, im nicht befüllten Zustand das elastische, poröse Übertragungsmittel 4 gegen die Membran 10. Die Poren sind dabei zusammengepresst. Beim Herausdrehen oder Entfernen des Deckels 16, 30, 40 relaxiert das elastische Übertragungsmittel 4 sofort und die auf der Oberfläche des Übertragungsmittels 4 befindliche Beschichtungslösung oder Beschichtungssuspension wird synchron zur Volumenvergrößerung des Übertragungsmittels 4 in die sich vergrößernden Poren eingesaugt.

Es ist erfindungsgemäß, dass im nicht befüllten Zustand der Deckel 16, 30, 40 über das Gewinde 18, 32 so in Richtung des elastischen, offen-porösen Übertragungsmittels 4 gedreht ist, dass dieses so zusammengepresst ist, dass aus einem großen Teil der Poren die Luft heraus gepresst ist.

Ein Verfahren kann also dadurch realisiert werden, dass in die Öffnung 20, 36 eines Schraubdeckels 16, 30, der das elastisch, offen-poröse Übertragungsmittel 4 zusammenpresst, die Beschichtungsflüssigkeit oder Beschichtungssuspension gefüllt wird und dass anschließend der Schraubdeckel 16, 30 aus dem Behälter 2 herausgedreht wird, wobei sich während des Herausdrehens des Schraubdeckels 16, 30 synchron das elastische, offen-poröse Übertragungsmittel 4 entspannt, sein Volumen vergrößert und dabei die Beschichtungslösung oder die Beschichtungssuspension in die sich ausdehnenden Poren aufsaugt. Der Schraubdeckel 16, 30 wird nach dem Herausdrehen verworfen.

Das Verfahren basiert auf der elastischen Rückstellkraft des elastischen, offen-porösen Übertragungsmittels 4. Der Vorteil des Verfahrens besteht darin, dass automatisch während des Herausdrehens des Schraubdeckels 16, 30 das Übertragungsmittel 4 mit der Beschichtungslösung oder Beschichtungssuspension befüllt wird. Ein umständliches Herauspressen der Luft aus den Poren ist nicht notwendig.

Im Folgenden werden Beispiele zur Herstellung einer Flüssigkeit für ein erfindungsgemäßes Verfahren ausgeführt.

Es werden 16,0 g Gentamicinsulfat (Fujian Fukang Ltd.) mit 4,0 ml pyrogenfreies steriles Wasser bei Raumtemperatur vermischt. Nach 24 Stunden Rühren bei Raumtemperatur mit einem Magnetrührer hat sich eine ölig-viskose gelbliche Lösung gebildet. Es ergibt sich eine Beschichtungslösung mit Gentamicinsulfat als Flüssigkeit zur Beschichtung eines medizinischen Implantats.

Herstellung einer Beschichtungslösung mit der Zweifachkombination Gentamicinsulfat und Clindamycinhydrochlorid:
Es werden 12,0 g Gentamicinsulfat (Fujian Fukang Ltd.), 4,0 g Clindamycinhydrochlorid (Sigma-Aaldrich) mit 4,0 ml pyrogenfreies steriles Wasser bei Raumtemperatur vermischt. Nach 24 Stunden Rühren bei Raumtemperatur mit einem Magnetrührer hat sich eine ölig-viskose gelbliche Lösung gebildet.

Herstellung einer Beschichtungslösung mit der Dreifachkombination Gentamicinsulfat, Clindamycinhydrochlorid und Vancomycinhydrochlorid:
Es werden 4,0 g Gentamicinsulfat (Fujian Fukang Ltd.), 4,0 g Clindamycinhydrochlorid (Sigma-Aaldrich) und 4,0 g Vancomycinhydrochlorid (Sigma-Aldrich) mit 8,0 ml pyrogenfreies steriles Wasser bei Raumtemperatur vermischt. Nach 24 Stunden Rühren bei Raumtemperatur mit einem Magnetrührer hat sich eine viskose gelbliche Lösung gebildet.

Mit konventionellen 10 ml Kunststoffspritzen werden jeweils 5 ml der Beschichtungslösungen der aufgeführten Beispiele aufgezogen und dann wird mit den befüllten Kunststoffspritzen 4 ml der jeweiligen Wirkstofflösung in eine erfindungsgemäße Vorrichtung gespritzt. Die Wirkstofflösung wird beim Bedienen der Komprimierungsvorrichtung beziehungsweise beim Entspannen beziehungsweise beim Relaxieren des Übertragungsmittels von dem porösen Übertragungsmittel aufgesaugt.

Anschließend werden übliche Zweymüller-Hüftprothesen kurz in eine befüllte erfindungsgemäße Vorrichtung kurz bis zum Ende des Schafts hineingetaucht sofort wieder heraus gezogen. Die Zweymüller-Hüftprothesen haben dann einen zähflüssigen Film der Wirkstofflösung an der Schaftoberfläche.

Die in der voranstehenden Beschreibung, sowie den Ansprüchen, Figuren und Ausführungsbeispielen offenbarten Merkmale der Erfindung können sowohl einzeln, als auch in jeder beliebigen Kombination für die Verwirklichung der Erfindung in ihren verschiedenen Ausführungsformen wesentlich sein.

### Bezugszeichenliste

- 1: Vorrichtung
- 2: Behälter
- 4: Übertragungsmittel / Schwamm
- 6: Öffnung / Schlitz im Übertragungsmittel
- 8: Hohlraum im Behälter
- 10: Membran
- 12,60: Haltering
- 14: Innengewinde
- 16, 30, 40: Deckel / Komprimierungseinrichtung
- 18, 32: Außengewinde
- 20, 36, 46: Durchführung
- 22: Membran
- 24, 38, 48: Trennwand
- 28,34: Flügel
- 42: Deckelwandung
- 50: Rippe
- 62: Ring
- 64: Haltevorrichtung

## Patentansprüche

1. Verfahren zur zumindest bereichsweisen Beschichtung eines medizinischen Implantats, vorzugsweise eines künstlichen Gelenks oder einer Fixierung für ein Gelenk, **dadurch gekennzeichnet, dass**
ein medizinisches Implantat bereitgestellt wird,
eine Flüssigkeit umfassend zumindest eine pharmazeutisch aktive Substanz in eine Vorrichtung (1) umfassend ein zumindest bereichsweise komprimiertes, elastisches, poröses Übertragungsmittel (4) eingefüllt wird,
das Volumen des Übertragungsmittels (4) vergrößert wird, wobei die Flüssigkeit zumindest teilweise in die Poren des Übertragungsmittels (4) aufgenommen wird und
anschließend die Flüssigkeit vom Übertragungsmittel (4) auf eine zu beschichtende Oberfläche des medizinischen Implantats übertragen wird.

2. Verfahren nach Anspruch 1, **dadurch gekennzeichnet, dass** das Volumen des Übertragungsmittels (4) durch Entspannen und/oder Dekomprimieren des Übertragungsmittels (4) vergrößert wird.

3. Verfahren nach einem der Ansprüche 1 oder 2, **dadurch gekennzeichnet, dass** das Übertragungsmittel (4) durch eine Komprimierungseinrichtung (16, 30, 40) in dem komprimierten Zustand gehalten wird, wobei durch eine Bedienung der Vorrichtung (1), insbesondere der Komprimierungseinrichtung (16, 30, 40), das Volumen des Übertragungsmittels (4) durch die Komprimierungseinrichtung (16, 30, 40) vergrößert wird.

4. Verfahren nach Anspruch 3, **dadurch gekennzeichnet, dass** als Komprimierungseinrichtung (16, 30, 40) ein Deckel (16, 30, 40) mit einem Gewinde (18, 32) verwendet wird, wobei das Gewinde (18, 32) in ein Gegengewinde (14) der Vorrichtung (1) greift, und dass durch Aufschrauben des Deckels (16, 30, 40) das Volumen des Übertragungsmittels (4) vergrößert wird

5. Verfahren nach einem der Ansprüche 1 bis 4, **dadurch gekennzeichnet, dass** die Flüssigkeit durch zumindest eine Durchführung (20, 36, 46) in der Vorrichtung (1), insbesondere im Deckel (16, 30, 40) der Vorrichtung (1) eingefüllt wird, insbesondere unmittelbar vor der Übertragung auf das medizinische Implantat, wobei vorzugsweise die zuvor geschlossene Durchführung (20, 36, 46) zuvor geöffnet wird, besonders bevorzugt indem eine Verschlussmembran (10) durchstoßen wird.

6. Verfahren nach einem der vorangehenden Ansprüche, **dadurch gekennzeichnet, dass**
die Flüssigkeit vom Übertragungsmittel (4) auf eine zu beschichtende Oberfläche des medizinischen Implantats übertragen wird, indem das medizinische Implantat durch einen Schlitz (6) oder mehrere Schlitze und/oder eine Ausnehmung (6) im Übertragungsmittel (4) geschoben und wieder herausgezogen wird, wobei vorzugsweise beim Herausziehen überschüssige Flüssigkeit abgestreift wird.

7. Verfahren nach Anspruch 6, **dadurch gekennzeichnet, dass** das Übertragungsmittel (4) beim Hindurchschieben des medizinischen Implantats zumindest bereichsweise komprimiert wird und dadurch die Flüssigkeit zumindest teilweise aus dem Übertragungsmittel (4) herausgedrückt wird und auf die Oberfläche des medizinischen Implantats übertragen wird, insbesondere allseitig auf die Oberfläche des medizinischen Implantats übertragen wird.

8. Verfahren nach einem der vorangehenden Ansprüche, **dadurch gekennzeichnet, dass**
ein Antibiotikum oder Antibiotika-Gemisch als Flüssigkeit verwendet wird, vorzugsweise eine Suspension oder eine Lösung, besonders bevorzugt eine wässrige Lösung.

9. Verfahren nach einem der vorangehenden Ansprüche, **dadurch gekennzeichnet, dass**
nach dem Übertragen der Flüssigkeit auf das medizinische Implantat ein Pulver auf die benetzte Oberfläche des medizinischen Implantats aufgebracht wird, bevorzugt das medizinische Implantat in ein Pulver eingetaucht wird, wobei das Pulver zumindest eine pharmazeutisch aktive Substanz umfasst.

10. Verfahren nach einem der vorangehenden Ansprüche, **dadurch gekennzeichnet, dass**
eine eingefärbte Flüssigkeit verwendet wird, so dass der beschichtete Bereich des medizinischen Implantats farblich kenntlich gemacht wird, wobei vorzugsweise die Vollständigkeit der Beschichtung des zu beschichtenden Bereichs anhand der Färbung geprüft wird.

11. Vorrichtung (1) zur zumindest bereichsweisen Beschichtung eines medizinischen Implantats, insbesondere mit einem Verfahren nach einem der vorangehenden Ansprüche, **dadurch gekennzeichnet, dass**
die Vorrichtung (1) zumindest ein zumindest bereichsweise komprimiertes, elastisches, poröses Übertragungsmittel (4) umfasst.

12. Vorrichtung (1) nach Anspruch 11, **dadurch gekennzeichnet, dass** die Vorrichtung (1) eine medizinische Beschichtungsvorrichtung (1) ist.

13. Vorrichtung (1) nach Anspruch 11 oder 12, **dadurch gekennzeichnet, dass** die Vorrichtung (1) eine Komprimierungseinrichtung (16, 30, 40) umfasst, wobei das Übertragungsmittel (4) durch die Komprimierungseinrichtung (16, 30, 40) in einem komprimierten Zustand haltbar ist und vorzugsweise durch Bedienen der Vorrichtung (1), insbesondere der Komprimierungseinrichtung (16, 30, 40) das Volumen des Übertragungsmittels (4) vergrößerbar ist.

14. Vorrichtung (1) nach Anspruch 13, **dadurch gekennzeichnet, dass** die Komprimierungseinrichtung (16, 30, 40) ein Deckel (16, 30, 40) mit einem Gewinde (18, 32) ist, der in die Vorrichtung (1) eingeschraubt ist, wobei das Volumen des Übertragungsmittels (4) durch Drehen und/oder Schrauben des Deckels (16, 30, 40) vergrößerbar ist.

15. Vorrichtung (1) nach Anspruch 14, **dadurch gekennzeichnet, dass** der Deckel (16, 30, 40) eine Durchführung (20, 36, 46) umfasst, durch die eine Flüssigkeit ins Innere der Vorrichtung (1) im Bereich des Übertragungsmittels (4) einbringbar ist, wobei die Durchführung (4) vorzugsweise in eine Verteilvorrichtung mündet, mit der die Flüssigkeit zumindest bereichsweise auf der Oberfläche des Übertragungsmittels (4) verteilbar ist und die Verteilvorrichtung besonders bevorzugt radial angeordnete Rippen (50) umfasst.

16. Vorrichtung (1) nach einem der Ansprüche 13 bis 15, **dadurch gekennzeichnet, dass**
zwischen der Komprimierungseinrichtung (16, 30, 40) und dem Übertragungsmittel (4) ein beweglicher Haltering (12, 60) angeordnet ist, der das zumindest eine Übertragungsmittel (4) im Inneren der Vorrichtung (1) hält, insbesondere unabhängig von der Komprimierung des Übertragungsmittels (4), wobei der Haltering (12, 60) vorzugsweise eine Arretierungsvorrichtung (64) umfasst, die mit einem Anschlag der Vorrichtung (1) den eingesetzten Haltering (12, 60) und damit das Übertragungsmittel (4) in der Vorrichtung (1) hält, besonders bevorzugt die Bewegung des Halterings (12, 60) in der Vorrichtung (1) begrenzt.

17. Vorrichtung (1) nach einem der Ansprüche 11 bis 16, **dadurch gekennzeichnet, dass**
das Übertragungsmittel (4) in einem Behälter (2) umfassend eine Öffnung zum Einführen und Herausnehmen des medizinischen Implantats angeordnet ist, wobei die Öffnung vorzugsweise durch den Deckel (16, 30, 40) geschlossen ist.

18. Vorrichtung (1) nach einem der Ansprüche 11 bis 17, **dadurch gekennzeichnet, dass**
die Vorrichtung (1) einen Abstreifer umfasst, der vorzugsweise im Bereich der Öffnung, insbesondere zwischen der Öffnung und dem Übertragungsmittel (4) angeordnet ist.

19. Vorrichtung (1) nach einem der Ansprüche 11 bis 18, **dadurch gekennzeichnet, dass**
das Übertragungsmittel (4) mindestens einen Einschnitt (6) umfasst, vorzugsweise mehrere Übertragungsmittel (4) jeweils zumindest einen Einschnitt (6) umfassen, wobei die Übertragungsmittel (4) derart zueinander angeordnet sind, dass die Einschnitte (6) zueinander versetzt angeordnet sind.

20. Vorrichtung (1) nach einem der Ansprüche 11 bis 19, **dadurch gekennzeichnet, dass**
das Übertragungsmittel (4) ein Schwamm ist oder die Übertragungsmittel (4) Schwämme sind, mit dem oder mit denen die Flüssigkeit auf die zu beschichtende Oberfläche des medizinischen Implantats übertragbar ist, wobei der Schwamm oder die Schwämme vorzugsweise aus einem Material mit hydrophiler Oberfläche gefertigt ist oder sind, wobei besonders bevorzugt zumindest ein anderes Teil der Vorrichtung, insbesondere der Abstreifer aus einem hydrophoben Material gefertigt sind.

21. Vorrichtung (1) nach einem der Ansprüche 11 bis 20, **dadurch gekennzeichnet, dass**
unterhalb des Übertragungsmittels (4) ein Hohlraum (8) angeordnet ist, wobei das Übertragungsmittel (4) vorzugsweise auf einer durchstoßbaren Membran (10) angeordnet ist, die das Innere des Hohlraums (8) abschließt.
